(19) Europäisches Patentamt
European Patent Office
Office européen des brevets

(11) EP 3 991 795 B1

(12) **EUROPEAN PATENT SPECIFICATION**

(45) Date of publication and mention of the grant of the patent:
**11.09.2024 Bulletin 2024/37**

(21) Application number: **20832373.3**

(22) Date of filing: **24.06.2020**

(51) International Patent Classification (IPC):
***A61K 31/706*** *(2006.01)* ***A61P 27/02*** *(2006.01)*
***A61P 29/00*** *(2006.01)*

(52) Cooperative Patent Classification (CPC):
**A61P 27/02; A61K 31/706; A61P 29/00**

(86) International application number:
**PCT/JP2020/024916**

(87) International publication number:
**WO 2020/262497 (30.12.2020 Gazette 2020/53)**

(54) **NOVEL USE OF NICOTINAMIDE MONONUCLEOTIDE (NMN) AND NICOTINAMIDE RIBOSIDE (NR)**

NEUARTIGE VERWENDUNG VON NICOTINAMID-MONONUKLEOTID (NMN) UND NICOTINAMID-RIBOSID (NR)

NOUVELLE UTILISATION DE NICOTINAMIDE MONONUCLÉOTIDE (NMN) ET DE NICOTINAMIDE RIBOSIDE (NR)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2019 JP 2019117642**

(43) Date of publication of application:
**04.05.2022 Bulletin 2022/18**

(73) Proprietors:
- **Senju Pharmaceutical Co., Ltd.**
  **Osaka-shi,**
  **Osaka 541-0048 (JP)**
- **Kyoto University**
  **Kyoto-shi, Kyoto 606-8501 (JP)**

(72) Inventors:
- **DOI, Masao**
  **Kyoto-shi, Kyoto 606-8501 (JP)**
- **NAKAJIMA, Takeshi**
  **Osaka-shi, Osaka 541-0048 (JP)**
- **MACHIDA, Mamiko**
  **Osaka-shi, Osaka 541-0048 (JP)**

(74) Representative: **Engelhard, Markus**
**Boehmert & Boehmert**
**Anwaltspartnerschaft mbB**
**Pettenkoferstrasse 22**
**80336 München (DE)**

(56) References cited:
**WO-A1-2020/054795 JP-A- 2008 542 296**
**JP-A- 2018 535 956 US-B2- 9 844 561**

- **BOLEKOVA A. ET AL: "Effect of retinoic acid on the nitrergic innervation of meibomian glands in rats", EUROPEAN JOURNAL OF HISTOCHEMISTRY: EJH, vol. 56, no. 4, 30 November 2012 (2012-11-30), Italy, pages 50, XP093051934, ISSN: 1121-760X, Retrieved from the Internet <URL:http://ejh.it/index.php/ejh/article/viewFile/2127/2180> DOI: 10.4081/ejh.2012.e50**
- **MILLS KATHRYN F ET AL: "Long-Term Administration of Nicotinamide Mononucleotide Mitigates Age-Associated Physiological Decline in Mice", CELL METABOLISM, CELL PRESS, UNITED STATES, vol. 24, no. 6, 27 October 2016 (2016-10-27), pages 795 - 806, XP029847468, ISSN: 1550-4131, DOI: 10.1016/J.CMET.2016.09.013**
- **MILLS, K.F. ET AL.: "Long-Term Administration of Nicotinamide Mononucleotide Mitigates Age-Associated Physiological Decline in Mice", CELL METABOLISM, vol. 24, 2016, pages 795 - 806, XP029847468, DOI: 10.1016/j.cmet.2016.09.013**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 991 795 B1

## Description

[Technical Field]

The present disclosure relates to a novel use of nicotinamide mononucleotide (NMN) and nicotinamide riboside (NR). For example, the present disclosure relates to a composition comprising NMN or NR for use in treating or preventing a disease accompanied by dysfunction of a meibomian gland or for use in treating or preventing meibomian gland dysfunction.

[Background Art]

In view of the suggested possibility of oxidized forms of nicotinamide adenine dinucleotide (NAD+) intermediate metabolites such as nicotinamide riboside (NR) and nicotinamide mononucleotide (NMN), i.e., enzymatic reaction products of nicotinamide phosphoribosyltransferase (NAMPT), exerting an effect of prolonging the lifespan, development thereof as a supplement is ongoing. However, the mechanism of action of the activity of NMN and NR still has not been completely understood.

BOLEKOVA A. ET AL: "Effect of retinoic acid on the nitrergic innervation of meibomian glands in rats",EUROPEAN JOURNAL OF HISTOCHEMISTRY: EJH, vol. 56, no. 4, 30 November 2012 (2012-11-30), page 50, indicates that retinoic acid could be suitable for the treatment of Meibomian gland dysfunction. T

[Summary of Invention]

[Solution to Problem]

The present invention is defined in the appended claims; any other disclosure in the present description is provided for referential purposes, only.

The present disclosure provides use of nicotinamide mononucleotide (NMN) and/or nicotinamide riboside (NR) or a composition comprising the same for improving the activity of Hsd3b6 or a homolog thereof. Improvement of the activity of Hsd3b6 or a homolog thereof can provide treatment or prevention of a disease, disorder, or symptom associated with the activity of Hsd3b6 or a homolog thereof.

The present disclosure particularly provides use of nicotinamide mononucleotide (NMN) and/or nicotinamide riboside (NR) or a composition comprising the same for improving the function of a meibomian gland. A composition can be characterized by being administered to an eye of a subject. Examples of improvement of a function of a meibomian gland include an increase in the number of meibomian gland acinar cells, increase in lipid secretion from a meibomian gland, and the like. In one embodiment, a composition for use in treating or preventing a disease accompanied by meibomian gland dysfunction can be provided. In another embodiment, the composition of the present disclosure exhibits an effect of improving meibomian gland tissue atrophy and can elevate synthase activity of a steroid that affects a lipid secreted from a meibomian gland. Since the composition of the present disclosure can normalize composition of lipids secreted from a meibomian gland, it is understood that lipids in the meibomian gland can be normalized. Furthermore, the composition of the present disclosure can promote production of testosterone, which is known to promote lipid production of a meibomian gland. Since sex hormones such as testosterone affects the lipid production of meibomian glands, it is understood that the composition of the present disclosure can normalize lipid component composition in meibomian gland tissue.

[Brief Description of Drawings]

[Figure 1] Figure 1 is a diagram showing atrophy of meibomian gland tissue associated with aging. At the left and center are pictures of stained eye lids of 6 month old and 24 month old mice, respectively. The right side is a bar graph that compares the mean stained areas for 6 month old and 24 month old mice.

[Figure 2] Figure 2 is a diagram showing the change in meibomian gland tissue of a 2 month old Hsd3b6 knockout (KO) mouse (size of meibomian gland and number of secretory ducts). The top row shows results for male mice and the bottom row shows results for female mice.

[Figure 3] Figure 3 is a diagram showing the change in Hsd3b6 enzymatic activity and proliferative activity of meibomian gland basal cells in a meibomian gland due to eye drop instillation of NMN or NR for 2 weeks into 1.5 year old wild-type mice.

[Figure 4] Figure 4 is a diagram showing the effect of NMN or NR eye drop instillation for 3 months into 1.75 year

old wild-type mice on meibomian gland tissue. The figure shows representative pictures from staining eye lids of the left eye (untreated) and right eye (eye drop instillation) and a graph showing the stained area in each individual (right eye/left eye).

[Figure 5] Figure 5 is a diagram showing the testosterone content in meibomian gland tissue after castration and after sham surgery on 2 month old wild-type (WT) and Hsd3b6 knockout (KO) male mice.

[Description of Embodiments]

The present disclosure is described hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

(Definitions)

As used herein, "about" refers to a range of $\pm$ 10% from the numerical value that is described subsequent to "about", unless noted otherwise.

As used herein, "subject" refers to the target of administration (transplantation) of a medicament or method for the treatment and prophylaxis of the present disclosure. Examples of subjects include mammals (e.g., humans, mice, rats, hamsters, rabbits, cats, dogs, cows, horses, sheep, monkeys, and the like), but primates are preferable and humans are particularly preferable.

As used herein, "or" is used when "at least one or more" of the listed matters in the sentence can be employed. When explicitly described herein as "within the range" of "two values", the range also includes the two values themselves.

As used herein, "therapy (treatment)" refers to healing of a disease or a symptom, or suppression of a symptom. "Prevention (prophylaxis)" refers to the prevention of expression of a disease or a symptom in advance. This concept also encompasses delaying the expression of a disease or symptom and minimizing the expression of a disease or symptom.

As used herein, "derivative" refers to a compound that has a core structure that is the same or similar to that of a parent compound, but has a chemical or physical modification such as a different functional group or an additional functional group. A derivative has the same or similar biological activity as the parent compound.

As used herein, "pharmaceutically acceptable salt" refers to an inorganic or organic acid addition salt of the compound of the present disclosure that is relatively nontoxic. These salts can be prepared temporarily during the final isolation and purification of a compound, or by reacting the purified compound in its free base form thereof separately with a suitable organic or inorganic salt, and isolating a salt thus formed.

Examples of pharmaceutically acceptable basic salts of the compound of the present disclosure include alkali metal salts such as sodium salts and potassium salts; alkaline earth metal salts such as calcium salts and magnesium salts; ammonium salts; aliphatic amine salts such as trimethylamine salts, triethylamine salts, dicyclohexylamine salts, ethanolamine salts, diethanolamine salts, triethanolamine salts, procaine salts, meglumine salts, diethanolamine salts, and ethylenediamine salts; aralkylamine salts such as N,N-dibenzylethylenediamine and benetamine salts; heterocyclic aromatic amine salts such as pyridine salts, picoline salts, quinoline salts, and isoquinoline salts; quaternary ammonium salts such as tetramethylammonium salts, tetraethylammonium salt, benzyltrimethylammonium salts, benzyltriethylammonium salts, benzyltributylammonium salts, methyltrioctylammonium salts, and tetrabutylammonium salts; basic amino acid salts such as arginine salts and lysine salts; and the like.

Examples of pharmaceutically acceptable acidic salts of the compound of the present disclosure include inorganic acid salts such as hydrochlorides, sulfates, nitrates, phosphates, carbonates, hydrogen carbonates, and perchlorates; organic acid salts such as acetates, propionates, lactates, maleates, fumarates, tartrates, malates, citrates, and ascorbates; sulfonates such as methanesulfonates, isethionates, benzenesulfonates, and p-toluenesulfonates; acidic amino acids such as aspartates and glutamates; and the like.

As used herein, "solvate" refers to a solvate of the compound of the present disclosure or a pharmaceutically acceptable salt thereof, encompassing, for example, a solvate of an organic solvent (e.g., alcohol (ethanol or the like)-ate), hydrate, and the like. When forming a hydrate, this can be coordinated with any number of water molecules. Examples of hydrates include monohydrates, dihydrates, and the like.

As used herein, "homolog" refers to a protein or gene having an amino acid sequence or a base sequence derived from the same ancestor. A homolog from speciation is referred to as an ortholog. A homolog newly created by

genetic duplication in an organism species is referred to as a paralog.

(Active ingredient)

Nicotinamide mononucleotide (NMN) is a compound with the following structure

[Chemical Formula 1]

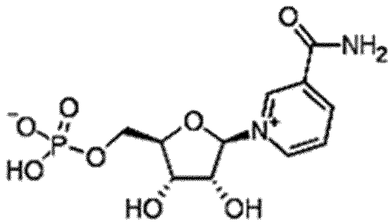

,

which is a nucleotide derived from nicotinamide and ribose. It is known as a biochemical progenitor of NAD+ (nicotinamide adenine dinucleotide).

Nicotinamide riboside (NR) is a compound with the following structure

[Chemical Formula 2]

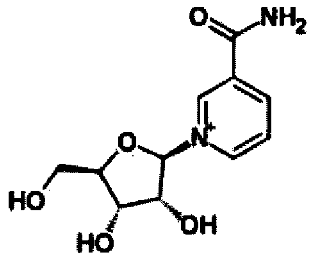

,

which is a conjugate of ribose and nicotinamide. This can be considered as NMN with a nucleotide replaced with ribose.

The present disclosure can provide a composition comprising NMN and/or NR. NMN and/or NR may be used as a pharmaceutically acceptable salt thereof. The Examples herein show that NMN and NR can improve Hsd3b6 enzymatic activity (or enzymatic activity of a homolog thereof such as HSD3B1). The Examples herein also show that NMN and NR can restore meibomian gland tissue. In one embodiment, a composition can comprise NMN. In one embodiment, a composition can comprise NR.

NMN and/or NR can be used in combination with an additional active ingredient. Examples of additional active ingredient include, but are not limited to, an antiinflammatory agent, antimicrobial drug, nutritional supplement, antioxidant, and the like.

(Disease)

The present disclosure can provide a composition for use in or method for improving activity of Hsd3b6 or a homolog thereof with NMN and NR. Hsd3b6 is directly controlled by the circadian clock, and a product of this gene is an important element of an aldosterone production pathway. In addition, a composition for use in or method for treating or preventing a disease, disorder, or symptom associated with activity of Hsd3b6 or a homolog thereof can be provided. Examples of the disease associated with activity of Hsd3b6 or a homolog thereof include, but are not limited to, meibomian gland dysfunction, diseases due to reduced steroid hormone (e.g., testosterone, estrogen, or the like), postmenopausal syndrome, osteoporosis, life style diseases, androgen deficiency in aging male, myocardial infarction, and the like.

In particular, the present disclosure can provide a composition for use in or method for improving a function of a meibomian gland with NMN and NR. A meibomian gland is one of the sebaceous glands that are at the edge of an eyelid. A meibomian gland prevents evaporation of the tear film of an eye, prevents tears from falling onto the cheek, and secretes an oily substance (sebum) which functions to seal the inside of a closed eyelid. A meibomian gland is also

known as a tarsal gland. Meibomian gland dysfunction (MGD) is defined as being accompanied by chronic ocular discomfort while having diffuse abnormality in the function thereof. The cause thereof is understood to be meibomian lipid denaturation or stagnation, chronic infection, or hyperkeratosis of inflamed ductal epithelium. The Examples of the present disclosure demonstrate that NMN and NR can restore the Hsd3b6 activity in a meibomian gland, and can increase the size of a meibomian gland. Examples of improvement of a function of a meibomian gland include an increase in the number of meibomian gland acinar cells, increase in lipid secretion from a meibomian gland, and treatment or prevention of a disease accompanied by dysfunction of a meibomian gland.

One embodiment of the present disclosure can provide a composition for use in or method for treating or preventing a disease accompanied by dysfunction of a meibomian gland with NMN and NR. For example, a composition for use in treating or preventing meibomian gland dysfunction, comprising nicotinamide mononucleotide (NMN) and/or nicotinamide riboside (NR) can be provided. Meibomian gland dysfunction can be accompanied by reduced meibum secretion. Meibomian gland dysfunction can be accompanied by an inflammatory disease. The inflammatory disease is, for example, at least one selected from the group consisting of meibomitis, superficial (punctate) keratitis, and blepharitis. Meibomian gland dysfunction can be accompanied by excessive lipid accumulation in a duct. Meibomian gland dysfunction can be accompanied by ocular discomfort, sensation of a foreign object, and/or sense of pressure. For example, the disease accompanied by meibomian gland dysfunction can be, but is not limited to, a disease selected from the group consisting of meibomitis, posterior blepharitis, keratitis, conjunctivitis, meibomitis-related keratoconjunctivitis, visual function disorder, androgen deficiency, atopic dermatitis, prostatic hyperplasia, ocular pemphigoid, discoid lupus (erythematosus), ectodermal dysplasia syndrome (anhidrotic ectodermal dysplasia), bone marrow transplant, hypertension, menopause, Parkinson’s disease, plaque psoriasis, rosacea, Sjogren’s syndrome, Stevens-Johnson syndrome, toxic epidermal necrolysis, Tuner syndrome, graft-versus-host disease (GVHD), and side effects due to drug therapy (retinoid, anti-androgen, antidepressant, antihistamine drug, or postmenopausal estrogen therapy). It can be understood that the composition of the present disclosure can improve the meibomian gland function itself so that the composition can be effectively used on any of the diseases described above.

Another embodiment of the present disclosure can provide a composition for use in or method for normalizing composition of lipids secreted from a meibomian gland and normalizing lipids in the meibomian gland. Since the composition of the present disclosure exhibits an effect of improving meibomian gland tissue atrophy and enhances synthase activity of steroid that affects lipids secreted from a meibomian gland, it is understood that the composition contributes to normalization of lipids in a meibomian gland. The method of measuring a lipid component in a meibomian gland is not particularly limited. Various methods that are well known in the art can be used, as long as the method can evaluate a lipid component in a meibomian gland.

In one embodiment of the present disclosure, the composition of the present disclosure elevates Hsd3b6 enzymatic activity. Thus, production of testosterone, which is known to promote lipid production of a meibomian gland, can be promoted. Since sex hormones such as testosterone affect lipid production in a meibomian gland, it is understood that the composition of the present disclosure contributes to normalization of lipid component composition in meibomian gland tissue by promoting production of sex hormones including testosterone via elevation of Hsd3b6 enzymatic activity.

(Formulation)

The composition of the present disclosure can be formulated into a suitable dosage form. For example, the composition of the present disclosure, when used as an ophthalmic composition, can be provided as an ophthalmic injection, ophthalmic ointment, eye drops, or ophthalmic perfusate. The composition can be formulated into any dosage form such as aerosol, liquid agent, extract, elixir, capsule, granule, pill, ointment, powder, tablet, solution, suspension, or emulsion. The composition can comprise any pharmaceutically acceptable additive and/or excipient that is known in the art. Examples of additives include, but are not limited to, tonicity adjusting agent, buffer, preservative, cosolvent, and thickener. For example, an ophthalmic composition can be provided in a form of a liquid agent prepared by dissolving an active ingredient into an aqueous solvent (e.g., water).

The composition of the present disclosure can be administered through any suitable route determined by those skilled in the art. The composition can be formulated to be suitable for administration through a route of administration selected from, but is not limited to, ocular injection, topical application (including application to an eye), eye drop, intravenous injection, intravenous drip, oral administration, parenteral administration, transdermal administration, and the like.

Examples of isotonizing agents include saccharides such as glucose, trehalose, lactose, fructose, mannitol, xylitol, and sorbitol, multivalent alcohols such as glycerol, polyethylene glycol, and propylene glycol, inorganic salts such as sodium chloride, potassium chloride, and calcium chloride, and the like. The amount thereof is preferably 0 to 5% by weight with respect to the entire amount of the composition.

Examples of chelating agents include edentates such as disodium edetate, disodium calcium edetate, trisodium edetate, tetrasodium edetate, and calcium edetate, ethylenediamine tetraacetate, nitrilotriacetic acid or a salt thereof,

sodium hexametaphosphate, citric acid, and the like. The amount thereof is preferably 0 to 0.2% by weight with respect to the entire amount of the composition.

Examples of stabilizers include sodium hydrogen sulfite and the like. The amount thereof is preferably 0 to 1% by weight with respect to the entire amount of the composition.

Examples of pH modifiers include acids such as hydrochloric acid, carbonic acid, acetic acid, and citric acid, as well as alkali metal hydroxides such as sodium hydroxide and potassium hydroxide, alkali metal carbonates or bicarbonates such as sodium carbonate, alkali metal acetate such as sodium acetate, alkali metal citrate such as sodium citrate, bases such as trometamol, and the like. The amount thereof is preferably 0 to 20% by weight with respect to the entire amount of the composition.

Examples of preservatives include sorbic acid, potassium sorbate, parahydroxybenzoate esters such as methyl parahydroxybenzoate, ethyl parahydroxybenzoate, propyl parahydroxybenzoate, and butyl parahydroxybenzoate, quaternary ammonium salts such as chlorhexidine gluconate, benzalkonium chloride, benzethonium chloride, and cetylpyridinium chloride, alkylpolyaminoethylglycine, chlorobutanol, polyquad, polyhexamethylene biguanide, chlorhexidine, and the like. The amount thereof is preferably 0 to 0.2% by weight with respect to the entire amount of the composition.

Examples of antioxidants include sodium hydrogen sulfite, dried sodium sulfite, sodium pyrosulfite, concentrated mixed tocopherol, and the like. The amount thereof is preferably 0 to 0.4% by weight with respect to the entire amount of the composition.

Examples of solubilizing agents include sodium benzoate, glycerin, D-sorbitol, glucose, propylene glycol, hydroxypropyl methylcellulose, polyvinylpyrrolidone, macrogol, D-mannitol, and the like. The amount thereof is preferably 0 to 3% by weight with respect to the entire amount of the composition.

Examples of thickening agents include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, polyvinyl alcohol, and the like. The amount thereof is desirably 0 to 70% by weight with respect to the entire amount of the composition.

Eye drops can be prepared by, for example, by dissolving or suspending the desired component described above in an aqueous solvent such as sterile purified water, saline, or buffer (e.g., borate buffer, phosphate buffer, etc.) or a non-aqueous solvent such as vegetable oil such as cottonseed oil, soybean oil, sesame oil, or peanut oil, adjusting the osmotic pressure to predetermined osmotic pressure, and applying sterilization such as mechanical sterilization.

An ophthalmic ointment can comprise an ointment base in addition to the various components described above upon preparation. Examples of the ointment base include, but are not particularly limited to, oily bases such as petroleum jelly, liquid paraffin, and polyethylene, emulsion bases prepared from emulsifying an oil phase and an aqueous phase with a surfactant or the like, water soluble bases consisting of hydroxypropyl methylcellulose, carboxymethyl cellulose, polyethylene glycol, and the like.

The composition, therapeutic agent, or prophylactic agent of the present disclosure can be provided as a kit. In a specific embodiment, the present disclosure provides a drug pack or kit comprising one or more containers filled with one or more components of the composition or medicament of the present disclosure. Optionally, information indicating approval for manufacture, use, or sale for human administration by a government agency regulating the manufacture, use, or sale of drugs or biological products can be displayed on such containers in a form specified by the government agency.

As used herein, "kit" refers to a unit providing parts to be provided (e.g., therapeutic drug, prophylactic drug, each component thereof, user manual, and the like) which are generally separated into two or more segments. Such a kit form is preferred when providing a composition, which should not be provided in a mixed state for stability or the like and is preferably used by mixing immediately prior to use. Such a kit advantageously comprises an instruction or user manual describing how the provided parts (e.g., therapeutic drug or prophylactic drug) are used or how the reagents should be processed. When a kit herein is used as a reagent kit, the kit generally comprises an instruction or the like describing the method of use of the therapeutic agent, prophylactic agent, or the like.

As used herein, "instruction" is a document with an explanation of the method of use of the present disclosure for physicians or other users. The instruction provides description for the method of detection of the present disclosure, how to use a diagnostic drug, or instruction for administration of a medicament or the like. The instruction may have a description instructing administration to the eye as a site of administration (e.g., by eye drop instillation, ophthalmic ointment, injection, etc.). The instruction is prepared in accordance with a format specified by a regulatory authority of the country in which the present disclosure is practiced (e.g., Ministry of Health, Labour and Welfare in Japan, Food and Drug Administration (FDA) in the U.S., or the like), with an explicit description showing approval by the regulatory authority. An instruction is a so-called package insert. An instruction is generally provided in, but not limited to, paper media. An instruction can also be provided in a form such as electronic media (e.g., web sites provided on the Internet or emails).

One embodiment can provide an eye drop comprising nicotinamide mononucleotide (NMN), wherein the nicotinamide mononucleotide (NMN) is contained at a concentration of 0.001 ug/ml or greater, 0.01 ug/ml or greater, 0.1

ug/ml or greater, 1 ug/ml or greater, 10 ug/ml or greater, 100 ug/ml or greater, 200 ug/ml or greater, 500 ug/ml or greater, 1 mg/ml or greater, 2 mg/ml or greater, 5 mg/ml or greater, 10 mg/ml or greater, or 50 mg/ml or greater.

One embodiment can provide an eye drop comprising nicotinamide riboside (NR), wherein the nicotinamide riboside (NR) is contained at a concentration of 0.001 ug/ml or greater, 0.01 ug/ml or greater, 0.1 ug/ml or greater, 1 ug/ml or greater, 10 ug/ml or greater, 100 ug/ml or greater, 200 ug/ml or greater, 500 ug/ml or greater, 1 mg/ml or greater, 2 mg/ml or greater, 5 mg/ml or greater, 10 mg/ml or greater, or 50 mg/ml or greater.

One embodiment can provide an ophthalmic ointment comprising nicotinamide mononucleotide (NMN), wherein the nicotinamide mononucleotide (NMN) is contained at a concentration of 0.001 ug/ml or greater, 0.01 ug/ml or greater, 0.1 ug/ml or greater, 1 ug/ml or greater, 2 ug/ml or greater, 5 ug/ml or greater, 10 ug/ml or greater, 50 ug/ml or greater, 100 ug/ml or greater, 200 ug/ml or greater, or 500 ug/ml or greater.

One embodiment can provide an ophthalmic ointment comprising nicotinamide riboside (NR), wherein the nicotinamide riboside (NR) is contained at a concentration of 0.001 ug/ml or greater, 0.01 ug/ml or greater, 0.1 ug/ml or greater, 1 ug/ml or greater, 2 ug/ml or greater, 5 ug/ml or greater, 10 ug/ml or greater, 50 ug/ml or greater, 100 ug/ml or greater, 200 ug/ml or greater, or 500 ug/ml or greater.

Use of a formulation with a concentration at a predetermined amount or greater in accordance with the dosage form can be advantageous for delivery to a meibomian gland, which is at the back of an eyelid, unlike delivery on the eye ball.

(Dose)

In one embodiment, utilization methods of the present disclosure include, for example, an eye drop, but the methods are not limited thereto. Examples thereof include modes of administration (administration methods and dosage forms) such as ophthalmic ointment, injection into the anterior chamber, impregnation into a sustained release agent, subconjunctival injection, and systemic administration (oral administration and intravenous injection).

The concentration of NMN or NR used in the present disclosure is generally about 0.001 to 1000 μM (μmol/l), preferably about 0.01 to 300 μM, more preferably about 0.03 to 100 μM, and still more preferably about 0.1 to about 30 μM. For example, other concentration ranges are generally 0.01 nM to 100 μM, about 0.1 nM to 100 μM, about 0.001 to 100 μM, about 0.01 to 75 μM, about 0.05 to 50 μM, about 1 to 10 μM, about 0.01 to 10 μM, about 0.05 to 10 μM, about 0.075 to 10 μM, about 0.1 to 10 μM, about 0.5 to 10 μM, about 0.75 to 10 μM, about 1.0 to 10 μM, about 1.25 to 10 μM, about 1.5 to 10 μM, about 1.75 to 10 μM, about 2.0 to 10 μM, about 2.5 to 10 μM, about 3.0 to 10 μM, about 4.0 to 10 μM, about 5.0 to 10 μM, about 6.0 to 10 μM, about 7.0 to 10 μM, about 8.0 to 10 μM, about 9.0 to 10 μM, about 0.01 to 50 μM, about 0.05 to 5.0 μM, about 0.075 to 5.0 μM, about 0.1 to 5.0 μM, about 0.5 to 5.0 μM, about 0.75 to 5.0 μM, about 1.0 to 5.0 μM, about 1.25 to 5.0 μM, about 1.5 to 5.0 μM, about 1.75 to 5.0 μM, about 2.0 to 5.0 μM, about 2.5 to 5.0 μM, about 3.0 to 5.0 μM, about 4.0 to 5.0 μM, about 0.01 to 3.0 μM, about 0.05 to 3.0 μM, about 0.075 to 3.0 μM, about 0.1 to 3.0 μM, about 0.5 to 3.0 μM, about 0.75 to 3.0 μM, about 1.0 to 3.0 μM, about 1.25 to 3.0 μM, about 1.5 to 3.0 μM, about 1.75 to 3.0 μM, about 2.0 to 3.0 μM, about 0.01 to 1.0 μM, about 0.05 to 1.0 μM, about 0.075 to 1.0 μM, about 0.1 to 1.0 μM, about 0.5 to 1.0 μM, about 0.75 to 1.0 μM, about 0.09 to 35 μM, or about 0.09 to 3.2 μM, more preferably about 0.01 to 10 μM, about 0.1 to 3 μM, or about 0.1 to 1.0 μM, but the concentration range is not limited thereto.

When used as an eye drop, the concentration of the formulation can be determined based on about 1 to 10000-fold, preferably about 100 to 10000-fold, such as about 1000-fold of the effective concentration described above while taking dilution with lacrimal fluid or the like into consideration as well as toxicity. The concentration can also be set to a concentration in excess thereof. The concentration is, for example, about 0.01 μM (μmol/l) to 1000 mM (mmol/l), 0.03 μM to 1000 mM, about 0.1 μM to 300 mM, about 0.3 μM to 300 mM, about 1 μM to 100 mM, about 3 μM to 100 mM, about 10 μM to 100 mM, about 30 μM to 100 mM, about 0.1 μM to 30 mM, about 0.3 μM to 30 mM, about 1 μM to 30 mM, about 3 μM to 30 mM, about 1 μM to 10 mM, about 3 μM to 10 mM, about 10 μM to 1 mM, about 30 μM to 1 mM, about 10 μM to 10 mM, about 30 μM to 10 mM, about 100 μM to 10 mM, about 300 μM to 10 mM, about 10 μM to 100 mM, about 30 μM to 300 mM, about 100 μM to 300 mM, or about 300 μM to 300 mM, and can be about 1 mM to 10 mM or about 1 mM to 100 mM. The concentration range can be determined by appropriately combining these upper limits and lower limits.

The effect amount of the medicament of the present disclosure, which is effective in treating a specific disease, disorder or condition, can vary depending on the property of the disorder or condition, but can be determined with a standard clinical technology based on the descriptions herein by those skilled in the art. Furthermore, identification of the optimal dosage range can be optionally assisted by using an in vitro assay. Since the precise dose to be used in a compound can vary depending on the route of administration or severity of a disease or disorder, the dose should be determined in accordance with the judgment of a physician or status of each patient. The dosage is not particularly limited, but can be, for example, 0.001, 1, 5, 10, 15, 100, or 1000 mg/kg body weight per adminsitration or within a range of two of such values. The dosing interval is not particularly limited, but can be, for example, 1, 2, or 4 doses every 1,

7, 14, 21, or 28 days, or 1, 2, or 4 doses for every number of days in the range between any two of such values. The dosage, number of doses, dosing interval, dosing period, and dosing method can be appropriately selected depending on the patient's age or body weight, symptom, mode of administration, target organ, or the like. For example, the composition of the present disclosure can be used as an eye drop. Further, a therapeutic drug preferably comprises an active ingredient at a therapeutically effective amount or an amount effective to exert a desired action. The effective dose can be estimated from a dose-response curve obtained from an in vitro or animal model test system.

Administration of an active ingredient at a predetermined amount of higher can be advantageous for delivery to a meibomian gland, which is at the back of an eyelid, unlike delivery on the eye ball.

(General technology)

The molecular biological methodology, biochemical methodology, and microbiological methodology used herein are well known and conventionally used in the art, which are described, for example, in Sambrook J. et al. (1989). Molecular Cloning: A Laboratory Manual, Cold Spring Harbor and its 3rd Ed. (2001); Ausubel, F. M. (1987). Current Protocols in Molecular Biology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M. (1992). Short Protocols in Molecular Biology: A Compendium of Methods from Current Protocols in Molecular Biology, Greene Pub. Associates; Ausubel, F. M. (1995). Short Protocols in Molecular Biology: A Compendium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Gait, M. J. (1985). Oligonucleotide Synthesis: A Practical Approach, IRL Press; Gait, M. J. (1990). Oligonucleotide Synthesis: A Practical Approach, IRL Press; Eckstein, F. (1991). Oligonucleotides and Analogues: A Practical Approach, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T. (1996). Bioconjugate Techniques, Academic Press, Bessatsu Jikken Igaku [Experimental Medicine, Supplemental Volume*],* "Idenshi Donyu & Hatsugen Kaiseki Jikken Ho [Experimental Methods for Transgenesis & Expression Analysis]", Yodosha, 1997, or the like.

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

[Examples]

Examples of the present disclosure are described hereinafter. Biological samples or the like, where applicable, were handled in compliance with the standards enacted by the Ministry of Health, Labour and Welfare, Ministry of Education, Culture, Sports, Science and Technology, or the like and, where applicable, based on the Declaration of Helsinki or ethical codes prepared based thereon.

(Example 1: Atrophy of meibomian gland tissue associated with aging/meibomian gland tissue in Hsd3b6 KO mice)

[Summary]

This Example demonstrates atrophy of meibomian gland tissue associated with aging and the change in meibomian gland tissue in Hsd3b6 KO mice.

[Materials and Methods]

(1) Whole mount meibomian glands Herxheimer's staining protocol

Meibomian gland tissue in mice was stained in accordance with the following protocol.

As a preparation method of 100 mL of staining solution, a bottle of stock solution prepared by dissolving Sudan IV to saturate in 100% ethanol was left standing in advance, and 70 mL of as much of the supernatant, 20 mL of 10% NaOH, and 10 mL of ultrapure water were mixed, tumbled for 5 minutes, and centrifuged to allow powder to precipitate.

The supernatant was filtered with a 0.45 um filter and used as a staining solution.

The cervical vertebrae of mice was dislocated, and skin of the head including meibomian glands was sampled. The sample was immersed in 4% paraformaldehyde while keeping the sample flat with a drawing pin, and immobilized using a rotator at 4°C for 24 hours. The periphery of meibomian glands was trimmed. The meibomian glands were immersed in 50% ethanol by using a rotator at room temperature for 10 minutes, then immersed for 20 minutes in 70% ethanol, and immersed in a staining solution using a rotator at room temperature for 24 hours in a falcon tube. The meibomian glands were then immersed for 10 minutes in 70% ethanol, and washing was repeated several times to confirm that the meibomian glands were stained well. The meibomian glands were immersed for 24 hours in amino alcohol in glycerin prepared by mixing 3.75 mL of 80% amino alcohol with 8.25 mL glycerin. The meibomian glands were placed on a microscope slide and encapsulated with glycerin jelly. Glycerin jelly was prepared by first mixing 10 g of gelatin with 60 ml of ultrapure water and 70 ml of glycerin, expanding the gelatin, and adding glycerin after about one hour and mixing while heating.

(2) Analysis of area of meibomian gland stained with Sudan IV

Post-staining area analysis was performed by using Photoshop and ImageJ and determining a threshold value of color intensity while looking at and comparing with a stain image based on the area of portions with an intensity at or above a given threshold value.

(3) Mouse

A floxed mouse in which the Exon 2 region including a start codon of an Hsd3b6 gene of a mouse is flanked by loxP was prepared. The mouse was crossbred with a CAG-Cre transgenic mouse to prepare an Hsd3b6 KO mouse which could no longer express Hsd3b6.

A test was conducted after raising a two month old mouse for 10 days under normal conditions (normal humidity condition) for free-feeding/drinking environment in a 12 hour bright and 12 hour dark cycle with a humidity of 40 to 60% and room temperature of 25°C.

A 24 month old mouse was a 78 week old (18 month old) male C57BL/6J mouse purchased from OrientalBioService, Inc., which was raised for 6 months under normal conditions (normal humidity conditions). A 6 month old C57BL/6J mouse was a 6 week old mouse purchased and raised to 6 month old under normal conditions (normal humidity conditions).

[Results]

Meibomian gland tissue was stained in a 6 month old mouse and a 24 month old mouse in accordance with the protocol described above, and the stained areas were compared. The results are shown in Figure 1. Atrophy in meibomian gland tissue and progression in meibomian gland dysfunction (MGD) due to aging were observed (N = 7).

Figure 2 shows the size of meibomian gland tissue and the number of secretory ducts in a 2 month old Hsd3b6 KO mouse and wild-type mouse. Just like in aged mice, Hsd3b6 KO mice were also found to exhibit meibomian gland atrophy. Since there is no difference in the number of secretory ducts due to genotype, it was found that meibomian gland atrophy is not an abnormality in the process of generating ducts.

(Example 2: Experiment of eye drop instillation of NMN or NR into a 1.5 year old wild-type mouse for 2 weeks)

[Summary]

This Example demonstrates the change in Hsd3b6 enzymatic activity and proliferative activity of meibomian gland acinar basal cells in meibomian glands due to eye drop instillation of NMN or NR for 2 weeks into 1.5 year old wild-type mice.

[Materials and Methods]

(1) The following is the summary of the eye drop instillation experiment.

[Table 1]

| Animal | Compound | Dosage | Route of administration | Dosing period | Number of animals | Experiment |
|---|---|---|---|---|---|---|
| WT mice (1.5 year old) | Vehicle | - | Eye drop instillation (only right eye) | 2 weeks | 5 | Enzymatic activity (steroid synthesis activity) |
| | | | | | 5 | BrdU incorporation assay (cell proliferative activity) |
| | NMN | 5% (5 μL/eye, 6 times/day) | | | 6 | Enzymatic activity (steroid synthesis activity) |
| | | | | | 6 | BrdU incorporation assay (cell proliferative activity) |
| | NR | 5% (5 μL/eye, 6 times/day) | | | 5 | Enzymatic activity (steroid synthesis activity) |
| | | | | | 6 | BrdU incorporation assay (cell proliferative activity) |

(2) Measurement of meibomian gland Hsd3b6 enzymatic activity

The meibomian gland Hsd3b6 enzymatic activity in mice was measured in accordance with the following protocol.

◦Preliminary preparation

Hank's balanced salt solution (HBSS) was bubbled with 95% $CO_2$/5% $O_2$ for 1 hour or more, and dispensed in 2 mL tubes at 200 μL each. The lid was closed while filled with 95% $CO_2$/5% $O_2$. HBSS was warmed with a 37°C water bath. A reaction incubator was warmed to 37°C, and a micro tube mixer was installed therein.

◦Startup of measuring equipment

The main power of a flow liquid scintillation counter (Perkin Elmer, Radiomatic 625TR) was turned on, and the main power of HPLC (Waters, 2795 Separations Module) was turned on to perform setup. Mobile phase A was replaced with water, and mobile phase B was replaced with acetonitrile. Once a column (Kanto Chemical, LiChroCART 250-4, LiChrosphere 100RP-18, particle size: Spm, 4mm I.D. × 250 mm) was installed, 40% acetonitrile (ACN) (A: 60%, B: 40%) was flown at 0.7 mL/min. The column oven temperature at this time was set to 40°C.

◦Purification of $^3$H-pregnenolone

$^3$H-DHEA was added to 1 mL of 40% ACN contained in a 2 mL tube, and the mixture was tapped. A microtube was set on an HPLC holder, and single administration was selected. The peak of contaminant produced after 22 minutes was not collected. Once the peak of contaminant decreased at about 23 minutes, the peak of $^3$H-DHEA was then manifested, so that collection into a glass centrifuge tube was started after reaching 1/3 of c.p.m. of the value of the peak. About 2.5 mL was collected in total from 4 minutes of collection. The liquid inside the glass centrifuge tube was stirred with a vortex. 25 μL was subjected to the liquid scintillation counter, and 25 μL was added to 3 mL of clear sol. After confirming that a small amount of $N_2$ was being discharged from an exhaust nozzle, the liquid inside the glass centrifuge tube was exsiccated with nitrogen at 75°C using Taitec's incubator.

◦Preparation of substrate

After cooling the $^3$H-DHEA exsiccated with nitrogen to room temperature, 1 μL/4,000 c.p.m. (value from measuring 25 μL with a liquid scintillation counter) of propylene glycol was added, and the $^3$H-DHEA was dissolved by vortexing for 5 minutes.

4 μL/4,000 c.p.m. of PBS was added. The mixture was vortexed. 10 μL was subjected to a liquid scintillation counter to confirm that the count is above 400000 c.p.m. The mixture was diluted with PBS:propylene glycol = 4:1. 400000 c.p.m./10 μL hot solution (1), 87 μM DHEA in 10% EtOH/90% D-PBS (2), 1 mM Dutasteride in 50% DMSO/50% DW (3), and 1 mM Fadrozole in 50% DMSO/50% DW (4) were prepared and mixed at a ratio of (1) : (2) : (3) : (4) = 5:5:1:1 as a solution of a substrate. This was warmed with a 37°C water bath prior to use.

◦Enzymatic reaction and extraction

A meibomian gland of the upper eyelid of one of the eyes was cut out and placed on weighing paper, with the side exposing the meibomian gland on top. The sample was chopped up into 200 μm thickness with a tissue chopper. The chopped meibomian gland was transferred into HBSS. The substrate solution was gently vortexed, and 24 μL was added to HBSS. The mixture was pipetted.

The mixture was incubated for 30 minutes while shaking with a micro tube mixer installed inside a 37°C incubator. After 30 minutes, the mixture was centrifuged at room temperature for 1 minute at 700 g, and the meibomian gland tissue was allowed to precipitate. The entire amount of HBSS was transferred into a test tube to which 2 mL of ethyl acetate was dispensed. The mixture was vortexed for 10 seconds. When the reaction was stopped, the mixture was immediately transferred onto ice.

200 μL of HBSS warmed to 37°C was added to a 2 mL tube with a meibomian gland remaining therein. After gently vortexing the substrate solution, 24 μL was added to HBSS, and the mixture was pipetted. 2 μL of 100 mM $NAD^+$ in D-PBS was added. The mixture was incubated for 30 minutes while shaking with a micro tube mixer installed inside a 37°C incubator. The mixture was centrifuged at room temperature for 1 minute at 700 g, and the meibomian gland tissue was allowed to precipitate. The entire amount of HBSS was transferred into a test tube to which 2 mL of ethyl acetate was dispensed. The mixture was vortexed for 10 seconds. When the reaction was stopped, the mixture was immediately transferred onto ice.

◦Steroid extraction

The mixture was centrifuged at room temperature for 10 minutes at 1500 rpm. 1.6 mL of a layer of ethyl acetate at the top layer was collected and exsiccated with nitrogen at 75°C. When the liquid was completely evaporated, 500 μL of 40% ACN was added. The mixture was vortexed for 3 minutes. The liquid was transferred to MILLIPORE Ultra free-MC installed on a 2 mL tube and centrifuged at room temperature for 2 minutes at 12000 g. A glass centrifuge tube was prewashed with 500 μL of 40% ACN and vortexed for 2 minutes. After passing through the same column's filter, the mixture was centrifuged at room temperature for 4 minutes at 12000 g. The elute was mixed by tapping, and 10 μL was added to 3 mL of clear sol. The mixture was subjected to a liquid scintillation counter (measurement of recovery rate).

◦Measurement

The column was equilibrated under the initial conditions (until the baseline and pressure stabilized). Sample sets were created in the same number as the number of samples to be measured. Since analysis does not stabilize for the first column, 40% ACN was allowed to flow as a sample. The lid of a 2 mL tube to be measured was opened. The tube was covered with a thinly stretched Parafilm and set up for HPLC. Continuous analysis was started.

HPLC conditions ($^3$H-DHEA)
Flow rate: 0.7 mL/min
Column temperature: 40°C
Gradient
0 to 30 min: 40 to 50% ACN (0.3%/min)
30 to 35 min: 70% ACN
35 to 40 min: 100% ACN
40 to 50 min: 100 to 40% ACN (6%/min)
50 to 60 min: 40% ACN

◦Analysis

A chromatograph was opened with ProFSA. Smoothing was performed by setting the SDA (smoothing function)

level to 8. The peaks were displayed with Find Peaks and Locate Peaks. The Report Preview was copied and pasted onto Word. Data was retrieved to deduce the enzymatic activity by the following formula.

[Numeral 1]

$$\text{Product Area (c.p.m.)} \times \left[ \frac{400{,}000}{\text{Post-recovery liquid scintillation count} \times 100} \right]$$

Inverse of recovery rate

(3) Cell proliferation assay through BrdU incorporation staining

BrdU (100 mg/kg body weight, Sigma-Aldrich) was intraperitoneally injected 30 minutes prior to harvesting eyelid tissue from a mouse. An eyelid comprising meibomian glands was immobilized with 4% PFA and embedded in paraffin. Antigens were activated by pressurizing a 5 um thick segment for 2.5 minutes in 10 mM sodium citrate buffer (pH 6.0). After incubating the segment for 24 hours at 4°C by using an anti-BrdU antibody (1:1000 dilution; Rockland Inc.), the immune response was made visible with 3,3'-diaminobenzidine (DAB) using secondary antibody Envision+ System- HRP Labelled Polymer Anti-Rabbit (Dako). For each tissue segment, BrdU positive cells in the basal cell layer of a meibomian gland were counted on a digital microscope picture captured at 20× magnification. The length of the outer circumference of a meibomian gland acinus was measured with ImageJ software, and the total BrdU positive cell count was normalized with the length of the outer circumference. The BrdU positive cells were counted by using 16 segments per individual.

[Results]

Figure 3 shows the change in Hsd3b6 enzymatic activity in a meibomian gland and change in the BrdU positive cell count in meibomian gland basal cells due to eye drop instillation of NMN, NR, or a solvent thereof (Vehicle, phosphate buffered saline). Hsd3b6 enzymatic activity and BrdU positive cell count increased significantly due to NMN eye drop instillation. Similar increase in Hsd3b6 enzymatic activity was observed, and BrdU positive cell count also increased significantly in the NR eye drop instillation group. Specifically, it can be understood that Hsd3b6 enzymatic activity increases topically at a meibomian gland and the proliferative activity of meibomian gland basal cells increases due to NMN or NR eye drop instillation.

(Example 3: 3 month experiment of NMN or NR eye drop instillation into 1.75 year old wild-type mouse)

[Summary]

This Example demonstrates the effect of 3 month NMN or NR eye drop installation into a 1.75 year old wild-type mouse on the meibomian gland tissue volume.

[Materials and Methods]

(1) The following is the outline of the eye drop instillation experiment.

[Table 2]

| Animal | Compound | Dosage | Route of administration | Dosing period | Number of animals | Experiment |
|---|---|---|---|---|---|---|
| WT mouse (1.75 year old) | Vehicle | - | Eye drop instillation (only right eye) | 3 months | 8 | Lipid stained tissue observation (meibomian gland volume evaluation) |
| | NMN | 5% (5 μL/eye, 4 times/day) | | | 8 | |
| | NR | 5% (5 μL/eye, 4 times/day) | | | 10 | |

(2) Meibomian gland tissue in a mouse was stained through the Whole mount meibomian glands Herxheimer's staining in the same manner as the approach in Example 1.

[Results]

Figure **4** shows the change in meibomian gland volume due to eye drop instillation of NMN, NR, and solvent thereof (Vehicle, phosphate buffered saline) . A significant increase in the meibomian gland volume was observed for both NMN eye drop instillation group and NR eye drop instillation group.

(Example 4: 3 month experiment of NMN or NR eye drop instillation into 1.75 year old wild-type mouse)

[Summary]

This Example demonstrates the effect of 3 month NMN or NR eye drop instillation into 1.75 year old wild-type mouse on lipid components in meibomian glands.

[Materials and Methods]

(1) The eye drop instillation experiment is performed in the same manner as Example 3.
(2) Harvest of lipids from mouse meibomian glands and lipid analysis

Meibomian glands are harvested from an eyelid of a euthanized mouse. Two meibomian glands harvested from one of the eyes of each animal are each placed in a glass vial containing 1 mL of chloroform:methanol = 2:1 (v/v) solvent mixture for lipid extraction, and lipids are extracted. The extract is relocated into a new vial. The extract can be stored for several months at -20°C or lower by evaporating the solution under nitrogen. The extract is then dissolved in a suitable solvent, and lipid composition is analyzed by APCI.

[Results]

Lipid components in a meibomian gland are normalized in NMN or NR eye drop instillation groups compared to a control Vehicle (phosphate buffered saline) eye drop instillation group.

(Example 5: Quantification of testosterone in meibomian gland)

[Materials and Methods]

After homogenizing a meibomian gland resected from an eyelid with methanol/water (75:25, v/v), steroid contained in the homogenate was extracted into dichloromethane by using Isolute SLE+ cartridge (Biotage) and evaporated under nitrogen to obtain a residue. The residue was dissolved in 50 μL of methanol/5% acetic acid solution containing an Amplifex Keto reagent (SCIEX) with a concentration of 10 mg/mL and incubated for 1 hour at room temperature. The testosterone concentration was then found by mass spectrometry using a liquid chromatography electrospray ionization

tandem mass spectrometer (QTRAP 4500 LC-MS/MS System; SCIEX) (derivative SRM transition: 403→164), and normalized by the wet weight of the extracted meibomian gland.

[Results]

Figure **5** shows the amount of testosterone in meibomian gland tissue of wild-type and Hsd3b6 deficient mice. The testosterone contained topically in meibomian glands decreased about 50% in Hsd3b6 deficient mice compared to wild-type mice. Furthermore, testosterone detected topically at meibomian glands decreased to 10% or less in Hsd3b6 knockout mice with the gonad (testicle) resected. It can be understood therefrom that not only circulating testosterone supplied from the testes, but also the same level of testosterone production topical to the meibomian gland contribute at the meibomian glands.

Since Hsd3b6 enzymatic activity is increased by eye drop instillation of NMN or NR, it is understood that production of testosterone which is known to promote lipid production of meibomian glands is promoted, and lipid component composition in meibomian gland tissue is normalized.

(Note)

The present application claims priority to Japanese Patent Application No. 2019-117642 filed on June 25, 2019 with the Japan Patent Office.

[Industrial Applicability]

The present disclosure can be used in the field of medicine, pharmaceutical products, healthcare, biology, biochemistry, and the like.

## Claims

1. A composition comprising nicotinamide mononucleotide (NMN) and/or nicotinamide riboside (NR), for use in treating or preventing a disease accompanied by dysfunction of a meibomian gland.

2. A composition comprising nicotinamide mononucleotide (NMN) and/or nicotinamide riboside (NR), for use in treating or preventing meibomian gland dysfunction.

3. The composition for use of claim 2, wherein the meibomian gland dysfunction is accompanied by reduced meibum secretion.

4. The composition for use of claim 2, wherein the meibomian gland dysfunction is accompanied by an inflammatory disease.

5. The composition for use of claim 4, wherein the inflammatory disease comprises at least one selected from the group consisting of meibomitis, superficial (punctate) keratitis, and blepharitis.

6. The composition for use of claim 2, wherein the meibomian gland dysfunction is accompanied by excessive lipid accumulation in a duct.

7. The composition for use of claim 2, wherein the meibomian gland dysfunction is accompanied by ocular discomfort, sensation of a foreign object, and/or sense of pressure.

8. The composition for use of claim 1, wherein the disease accompanied by the dysfunction of a meibomian gland is meibomitis, posterior blepharitis, keratitis, conjunctivitis, meibomitis-related keratoconjunctivitis, ocular pemphigoid, Sjogren's syndrome, Stevens-Johnson syndrome, graft-versus-host disease (GVHD), and visual function disorder.

## Patentansprüche

1. Zusammensetzung, umfassend Nicotinamid-Mononukleotid (NMN) und/oder Nicotinamid-Ribosid (NR), zur Verwendung bei der Behandlung oder Vorbeugung einer Krankheit, die von einer Dysfunktion einer Meibom-Drüse

begleitet ist.

2. Zusammensetzung, umfassend Nicotinamid-Mononukleotid (NMN) und/oder Nicotinamid-Ribosid (NR), zur Verwendung bei der Behandlung oder Vorbeugung einer Meibom-Drüsen-Dysfunktion.

3. Zusammensetzung zur Verwendung nach Anspruch 2, wobei die Meibom-Drüsen-Dysfunktion von reduzierter Meibumsekretion begleitet wird.

4. Zusammensetzung zur Verwendung nach Anspruch 2, wobei die Meibom-Drüsen-Dysfunktion von einer Entzündungskrankheit begleitet wird.

5. Zusammensetzung zur Verwendung nach Anspruch 4, wobei die Entzündungskrankheit mindestens eine umfasst, die aus der Gruppe ausgewählt ist, bestehend aus Meibomitis, Keratitis superficialis punctata und Blepharitis.

6. Zusammensetzung zur Verwendung nach Anspruch 2, wobei die Meibom-Drüsen-Dysfunktion von exzessiver Lipidansammlung in einem Drüsengang begleitet wird.

7. Zusammensetzung zur Verwendung nach Anspruch 2, wobei die Meibom-Drüsen-Dysfunktion von Augenbeschwerden, einem Fremdkörpergefühl, und/oder einem Druckgefühl begleitet wird.

8. Zusammensetzung zur Verwendung nach Anspruch 1, wobei die Krankheit, die von der Dysfunktion einer Meibom-Drüse begleitet wird, Meibomitis, posteriore Blepharitis, Keratitis, Konjunktivitis, Meibomitis-bedingte Keratokonjuktivitis, okuläres Pemphigoid, Sjögren-Syndrom, Stevens-Johnson-Syndrom, Graft-versus-Host-Disease (GVHD), und eine Seheinschränkung ist.

## Revendications

1. Composition comprenant du nicotinamide mononucléotide (NMN) et/ou du nicotinamide riboside (NR) pour une utilisation dans le traitement ou la prévention d'une maladie s'accompagnant d'un dysfonctionnement d'une glande de Meibomius.

2. Composition comprenant du nicotinamide mononucléotide (NMN) et/ou du nicotinamide riboside (NR) pour une utilisation dans le traitement ou la prévention d'un dysfonctionnement d'une glande de Meibomius

3. Composition pour une utilisation selon la revendication 2, dans laquelle le dysfonctionnement de la glande de Meibomius s'accompagne d'une sécrétion réduite de meibum.

4. Composition pour une utilisation selon la revendication 2, dans laquelle le dysfonctionnement de la glande de Meibomius s'accompagne d'une maladie inflammatoire.

5. Composition pour une utilisation selon la revendication 4, dans laquelle la maladie inflammatoire comprend au moins une maladie choisie dans le groupe constitué par la meibomite, la kératite superficielle (ponctuée) et la blépharite.

6. Composition pour une utilisation selon la revendication 2, dans laquelle le dysfonctionnement de la glande de Meibomius s'accompagne d'une accumulation excessive de lipides dans un canal.

7. Composition pour une utilisation selon la revendication 2, dans laquelle le dysfonctionnement de la glande de Meibomius s'accompagne d'un inconfort oculaire, d'une sensation de corps étranger et/ou d'une sensation de pression.

8. Composition pour une utilisation selon la revendication 1, dans laquelle la maladie s'accompagnant du dysfonctionnement d'une glande de Meibomius est la meibomite, la blépharite postérieure, la kératite, la conjonctivite, la kératoconjonctivite liée à la meibomite, la pemphigoïde oculaire, le syndrome de Sjögren, le syndrome de Stevens-Johnson, la maladie du greffon contre l'hôte (MGCH) et le trouble de la fonction visuelle.

FIG.1

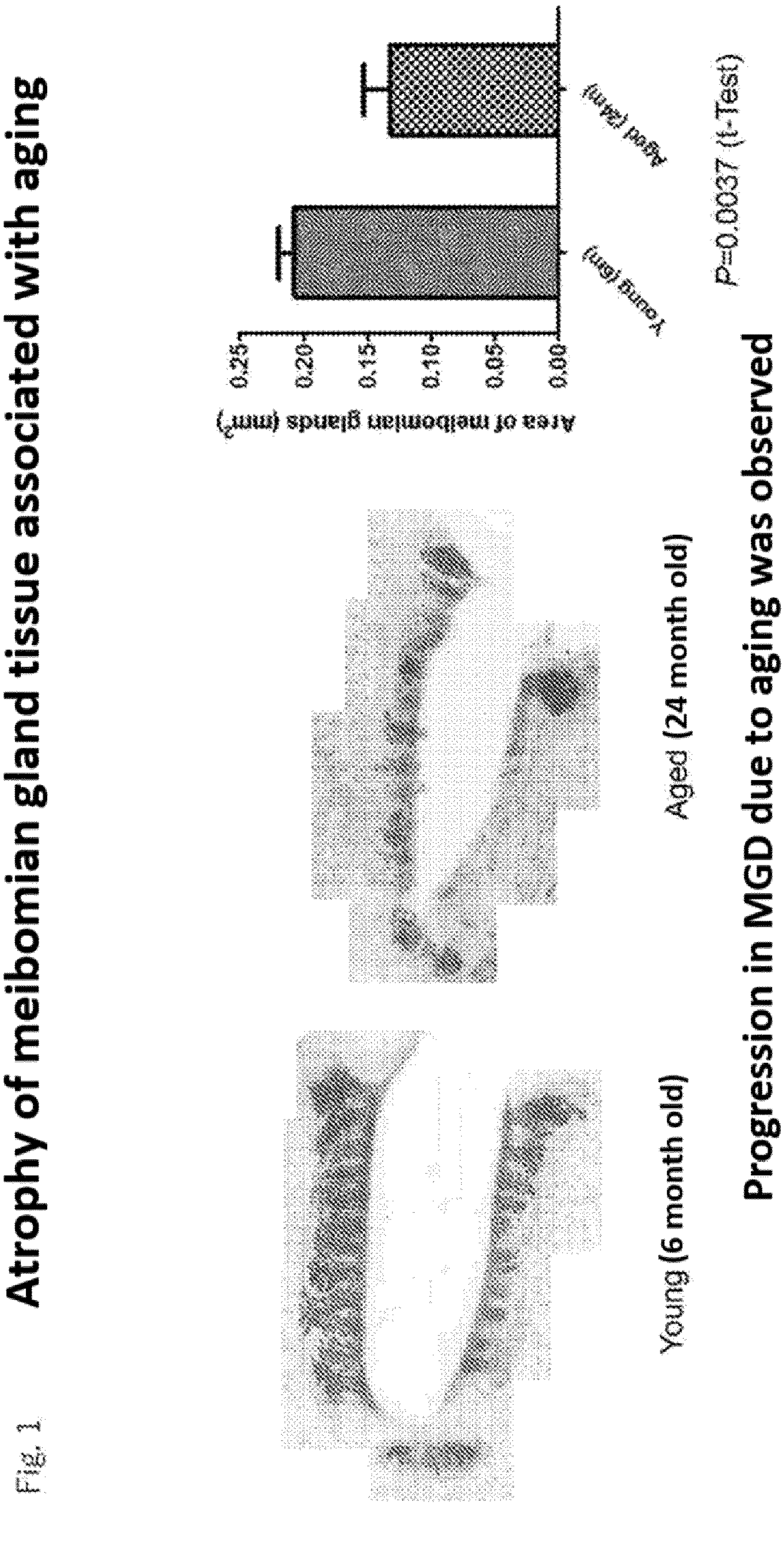
Fig. 1
Atrophy of meibomian gland tissue associated with aging
Young (6 month old)
Aged (24 month old)
Area of meibomian glands (mm²)
0.25
0.20
0.15
0.10
0.05
0.00
Young (6m)
Aged (24m)
P=0.0037 (t-Test)
Progression in MGD due to aging was observed

FIG.2

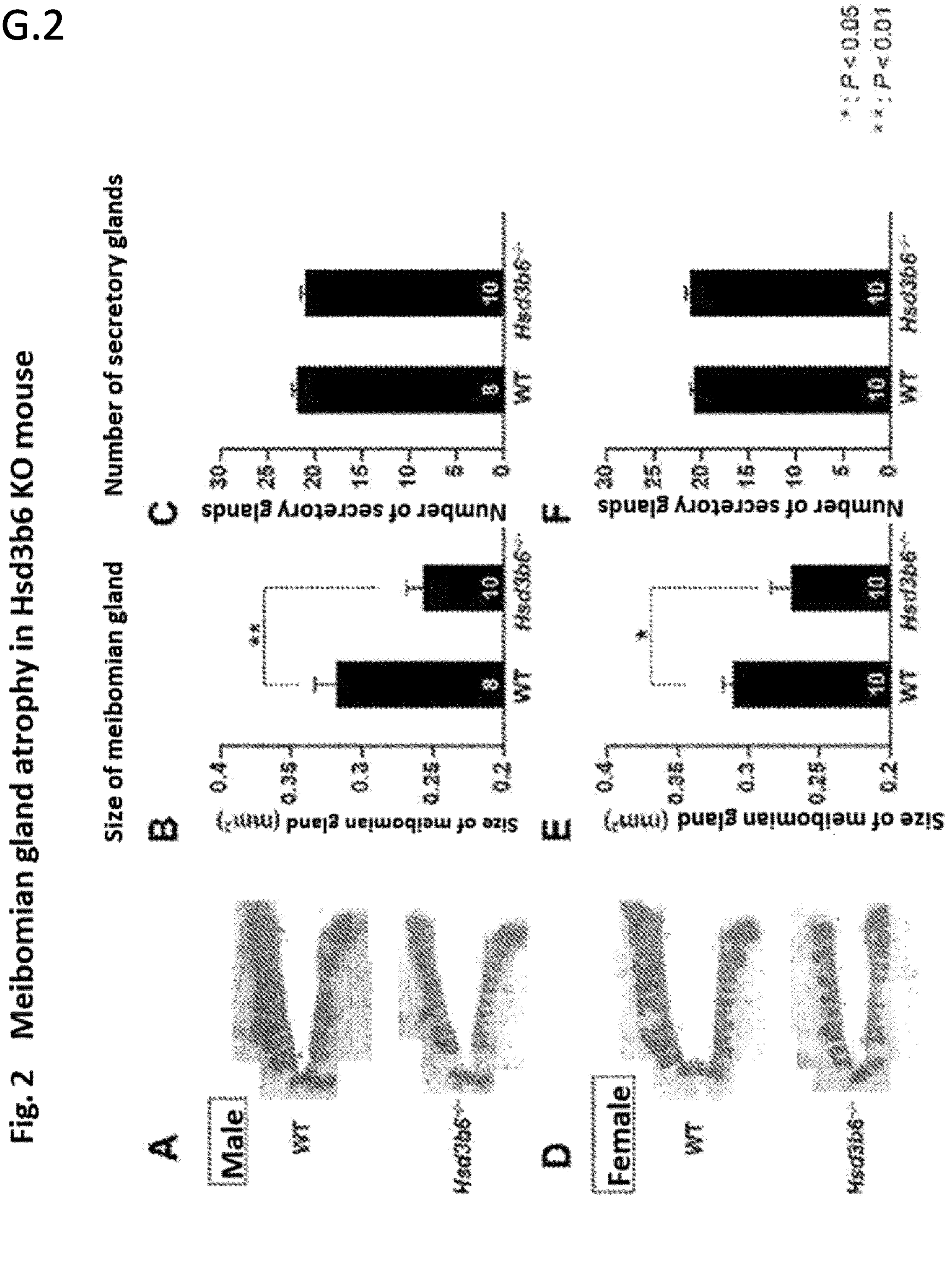
Fig. 2 Meibomian gland atrophy in Hsd3b6 KO mouse
A
Male
WT
Hsd3b6-/-
D
Female
WT
Hsd3b6-/-
Size of meibomian gland
B
Size of meibomian gland (mm²)
0.4
0.35
0.3
0.25
0.2
**
8
10
WT
Hsd3b6-/-
E
*
10
10
Number of secretory glands
C
Number of secretory glands
30
25
20
15
10
5
0
8
10
F
10
10
*; P < 0.05
**; P < 0.01

FIG.3

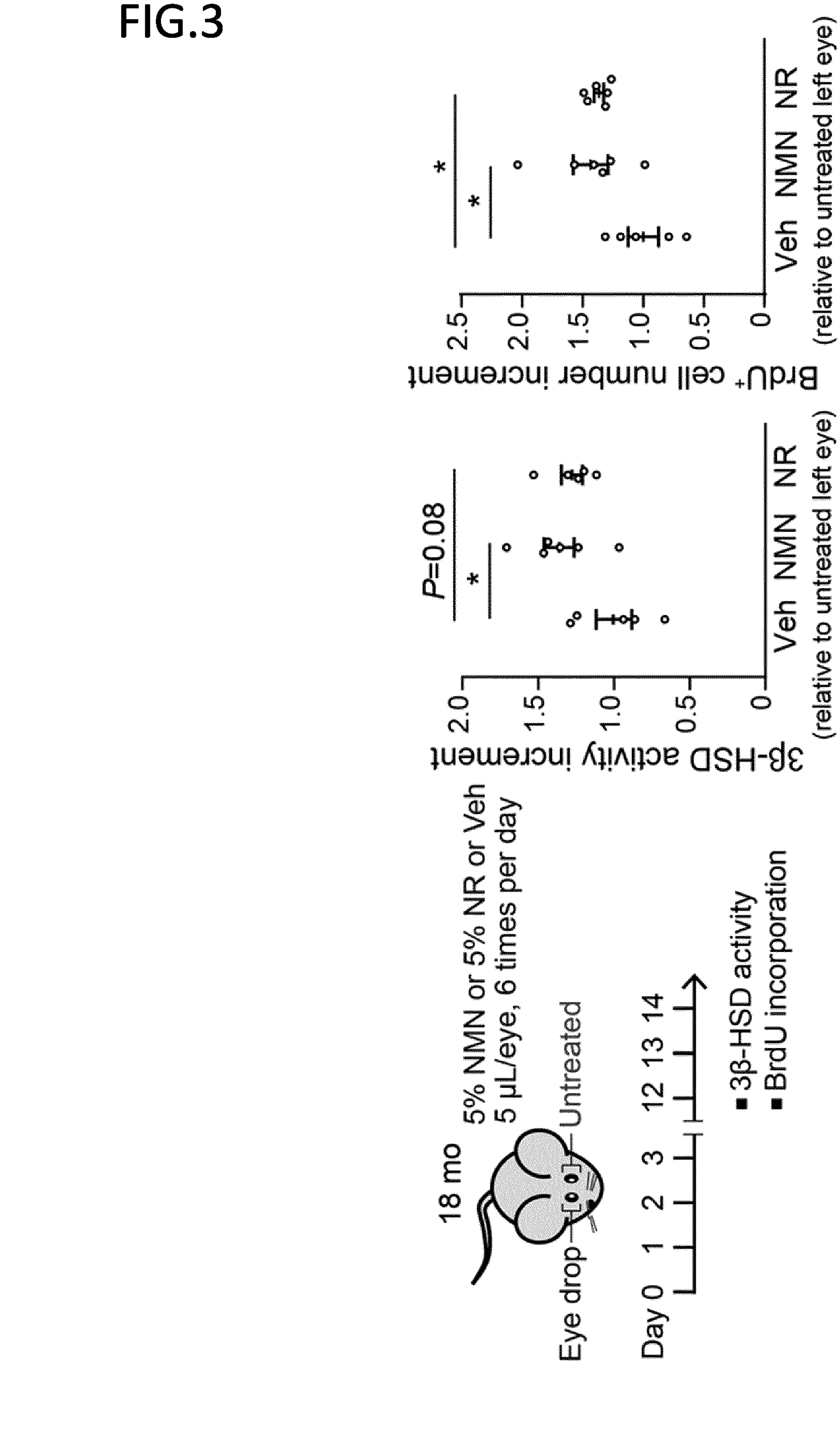
18 mo
5% NMN or 5% NR or Veh
5 µL/eye, 6 times per day
Eye drop
Untreated
Day 0 1 2 3 12 13 14
■ 3β-HSD activity
■ BrdU incorporation
3β-HSD activity increment
2.0 1.5 1.0 0.5 0
P=0.08
*
Veh NMN NR
(relative to untreated left eye)
BrdU+ cell number increment
2.5 2.0 1.5 1.0 0.5 0
*
*
Veh NMN NR
(relative to untreated left eye)

FIG.4

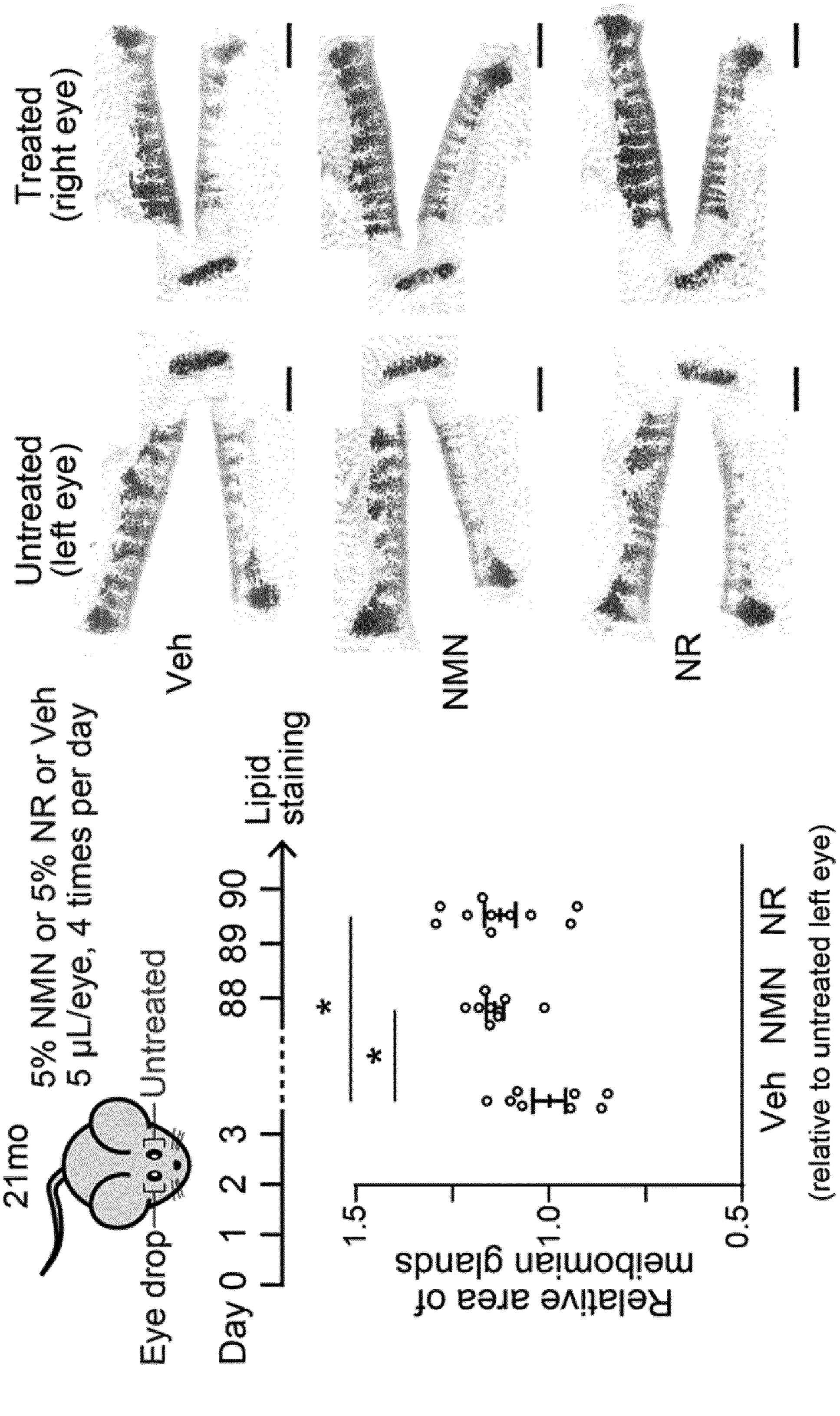

21mo
5% NMN or 5% NR or Veh
5 µL/eye, 4 times per day
Eye drop
Untreated
Day 0 1 2 3
88 89 90
Lipid staining
Relative area of meibomian glands
1.5
1.0
0.5
*
*
Veh NMN NR
(relative to untreated left eye)
Untreated (left eye)
Treated (right eye)
Veh
NMN
NR

FIG.5

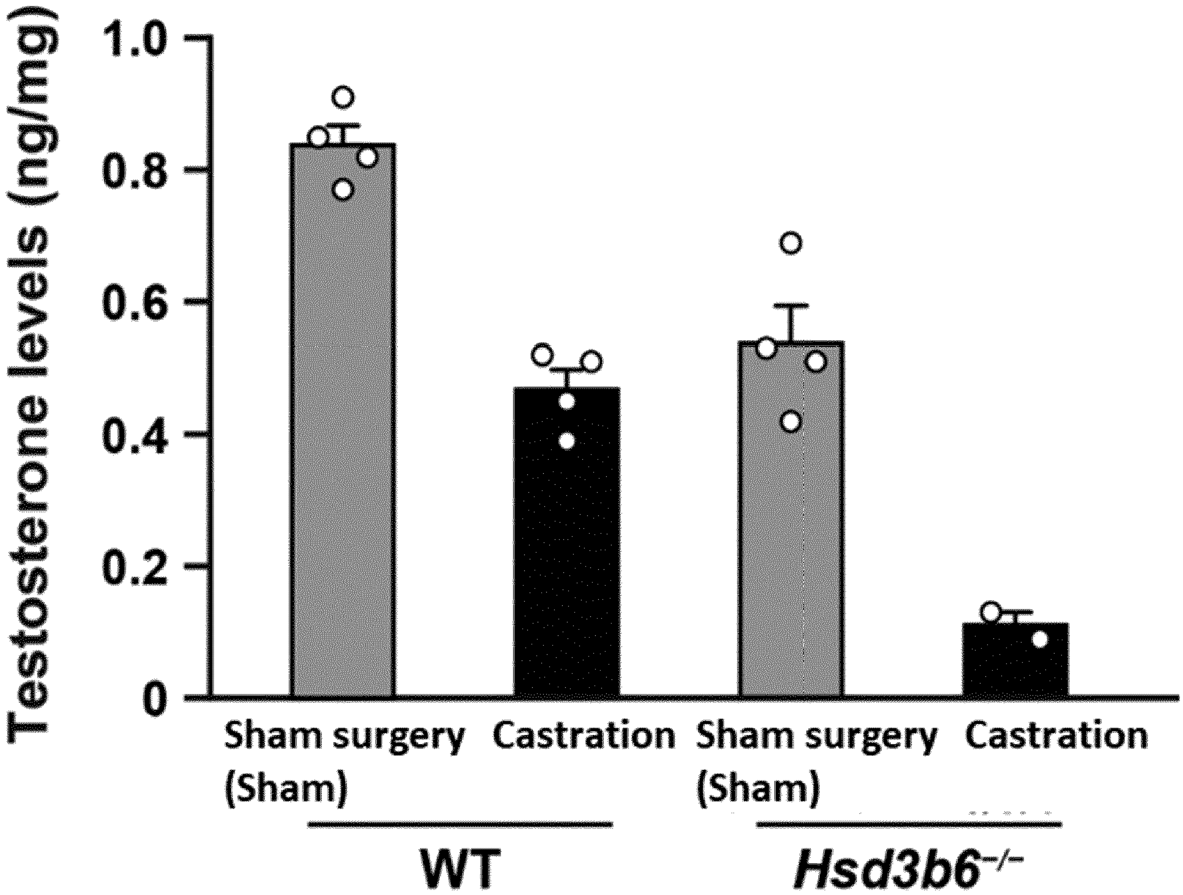
Testosterone levels (ng/mg)
1.0
0.8
0.6
0.4
0.2
0
Sham surgery (Sham)
Castration
Sham surgery (Sham)
Castration
WT
Hsd3b6-/-

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019117642 A


### Non-patent literature cited in the description


- **BOLEKOVA A. et al.** Effect of retinoic acid on the nitrergic innervation of meibomian glands in rats. *EUROPEAN JOURNAL OF HISTOCHEMISTRY: EJH,* 30 November 2012, vol. 56 (4), 50
- **SAMBROOK J. et al.** Molecular Cloning: A Laboratory Manual. Cold Spring Harbor, 1989
- **AUSUBEL, F. M.** Current Protocols in Molecular Biology. Greene Pub. Associates and Wiley-Interscience, 1987
- **AUSUBEL, F. M.** Short Protocols in Molecular Biology: A Compendium of Methods from Current Protocols in Molecular Biology. Greene Pub. Associates and Wiley-Interscience, 1989
- **INNIS, M. A.** PCR Protocols: A Guide to Methods and Applications. Academic Press, 1990
- **AUSUBEL, F. M.** Short Protocols in Molecular Biology: A Compendium of Methods from Current Protocols in Molecular Biology. Greene Pub. Associates, 1992
- **AUSUBEL, F. M.** Short Protocols in Molecular Biology: A Compendium of Methods from Current Protocols in Molecular Biology. Greene Pub. Associates, 1995
- **INNIS, M. A. et al.** PCR Strategies. Academic Press, 1995
- **AUSUBEL, F. M.** Short Protocols in Molecular Biology: A Compendium of Methods from Current Protocols in Molecular Biology. Wiley, 1999
- **SNINSKY, J. J. et al.** PCR Applications: Protocols for Functional Genomics. Academic Press, 1999
- **GAIT, M. J.** Oligonucleotide Synthesis: A Practical Approach. IRL Press, 1985
- **GAIT, M. J.** Oligonucleotide Synthesis: A Practical Approach. IRL Press, 1990
- **ECKSTEIN, F.** Oligonucleotides and Analogues: A Practical Approach. IRL Press, 1991
- **ADAMS, R. L. et al.** The Biochemistry of the Nucleic Acids. Chapman & Hall, 1992
- **SHABAROVA, Z. et al.** *Advanced Organic Chemistry of Nucleic Acids,* 1994
- **BLACKBURN, G. M. et al.** Nucleic Acids in Chemistry and Biology. Oxford University Press, 1996
- **HERMANSON, G. T.** Bioconjugate Techniques. Academic Press, 1996
- **BESSATSU JIKKEN IGAKU.** *Experimental Medicine*
- **IDENSHI DONYU ; HATSUGEN KAISEKI ; JIKKEN HO.** *Experimental Methods for Transgenesis & Expression Analysis,* 1997